# EUROPEAN PATENT APPLICATION

(11) **EP 4 374 855 A1**
(43) Date of publication of application: **29.05.2024**
(21) Application number: 23210907.4
(22) Date of filing: 20.11.2023
(51) Int. Cl.: A61K 9/20, A61K 31/4184

(54) **PHARMACEUTICAL DOSAGE FORM OF CANDESARTAN AND INDAPAMIDE**

(30) Priority: 22.11.2022 SI 202200230
(71) Applicant: KRKA, D.D., Novo Mesto, 8501 Novo mesto (SI)
(72) Inventor: Baric, Matej, 8501 Novo mesto (SI); Brec, Tina, 8501 Novo mesto (SI); Pohlen, Mitja, 8501 Novo mesto (SI); Kapele, Toma, 8501 Novo mesto (SI); Omerzu, Maja, 8501 Novo mesto (SI); Vrhunc, Ga per, 8501 Novo mesto (SI)
(74) Representative: Kutzenberger Wolff & Partner

(57) **Abstract**

The invention relates to an antihypertensive compound preparation, in particular to a solid pharmaceutical dosage form for oral administration comprising (i) an intragranular phase comprising candesartan cilexetil; and (ii) an extragranular phase comprising indapamide. The invention further relates to a process for preparing such pharmaceutical dosage form.

## Description

Priority is claimed of Slovenian patent application no. P-202200230 that was filed on November 22, 2022.

The invention relates to an antihypertensive compound preparation, in particular to a solid pharmaceutical dosage form for oral administration comprising (i) an intragranular phase comprising candesartan cilexetil; and (ii) an extragranular phase comprising indapamide. The invention further relates to a process for preparing such pharmaceutical dosage form.

Candesartan belongs to the class of benzimidazole-7-carboxylic acid. It is a selective AT1 subtype angiotensin II receptor antagonist, used for the treatment of cardiovascular diseases such as hypertension, heart failure and post myocardial infarction as disclosed in US 5,196,444.

Candesartan is poorly absorbed after oral administration and hence, its prodrug candesartan cilexetil was developed. Following oral administration candesartan cilexetil undergoes hydrolysis to form candesartan. Chemically, candesartan cilexetil is (±)-1-hydroxyethyl,2-ethoxy-1-[p-(1H-tetrazol-5-ylphenyl)benzyl]-7-benzimidazole carboxylate, cyclohexyl carbonate (ester).

Indapamide (4-chloro-N-(2-methylindolyn-1-yl)-3-sulfamoyl-benzamide) is a thiazide diuretic indicated in the treatment of hypertension and edema. Indapamide is known from e.g. US 3,565,911. Various processes for the preparation of indapamide and for pharmaceutical compositions comprising indapamide are known from the prior art.

Mono-products of candesartan and indapamide are present on the market, but there are no products combining both active ingredients in a single dosage form.

CN 1493284 A discloses an antihypertensive compound preparation characterized in that it contains 0.15-2.5 mg of a diuretic indapamide, 2-800 mg of an angiotensin II receptor antagonist selected from losartan, valsartan, irbesartan, candesartan, telimi sartan (telmisartan), eprosartan (eprosartan), olmesartan (olmesartan), or a mixture thereof, and a pharmaceutically acceptable carrier. Specific examples are given for combinations of indapamide with losartan, indapamide with valsartan, and indapamide with irbesartan. Most preferred is the combination of indapamide with losartan.

2351/CHE/2011 discloses a stable oral dosage comprising candesartan cilexetil, prepared by granulating a blend of excipients comprising adsorbent with a dispersion comprising candesartan and propylene glycol and one or more pharmaceutically acceptable excipients in a solvent. Combination of candesartan and a diuretic selected from amiloride, chlortalidone, furosemide, hydrochlorothiazide and indapamide is also disclosed. Example in description covers the combination of candesartan and hydrochlorothiazide.

Several studies have been done, which observed the effect of candesartan cilexetil combined with indapamide (as two separate tablets) in the treatment of hypertension.

Qiang, HE Yijian, Supplement Makes Industry Technological Modem Hospital, July 2008, Vol 8 relates to candesartan cilexetil combined with indapamide sustained-release tablets in the treatment of mild to moderate essential hypertension.

Xu Guozhong, Traffic Medicine 2010 Volume 24 Issue 3 Med Jof Communications, 2010, Vol. 24. No. 3 relates to Observation on the effect of candesartan cilexetil combined with indapamide in the treatment of hypertension.

Kong Lingmin, Chinese and Foreign Medical Research" No. 10, volume 9, 2012 March relates to the efficacy of candesartan and indapamide in the treatment of elderly patients with diabetes and hypertension.

Zhang Yongqing, Shen Jianning, Xu Qiuju; Article ID:1006-0979 ( 2013 ) 33-0029-02 relates to Efficacy observation of candesartan combined with indapamide in the treatment of hypertension.

Zhao Ping, Chinese and Foreign Health Digest, Volume 10, Issue 10, March 2013 World Health Digest Medical Periodical relates to Efficacy of candesartan combined with indapamide on essential hypertension and its effect on cardiac function.

Abdul Rehman Abdul Aini, The World's Latest Medical Information Digest, Vol.13, No.23, 2013 relates to Clinical efficacy of candesartan combined with indapamide in the treatment of diabetes mellitus with hypertension.

Ni Lin, Zhou Haimei, Bu Zhiping, J of Clinical Rational Drug Lse, April 2013,ol. 6 JNo. 4 A relates to Clinical observation of candesartan combined with indapamide in the treatment of diabetes complicated with hypertension.

Luo Youqin, Clinical Research Psychologist, Volume 24, Issue 23, August 2018 relates to Antihypertensive effect of candesartan combined with indapamide on diabetes complicated with hypertension.

The pharmaceutical formulations comprising candesartan or indapamide of the prior art and the methods for their preparation are not satisfactory in every respect and there is a demand for improved pharmaceutical formulations.

Conventional indapamide tablets are prepared by a wet granulation process or a direct compression process as disclosed in e.g. CN 110 538 160 A. The wet granulation process is the main method, but the wet granulation process is prone to the particle size of the raw and auxiliary materials affecting the indapamide dissolution rate. It can cause the problem that the dissolution of the dosage form may be too fast or too slow, it can be difficult to control the drug release amount, and impurities can easily be generated during the wet granulation process. One known impurity of indapamide is *"indapamide impurity A*", i.e. 2-methyl-1-nitroso-2,3-dihydroindole, as disclosed in CN 114 594 174 A.

It is an object of the invention to provide a solid pharmaceutical form comprising candesartan and indapamide. The dosage form should preferably provide conventional release of candesartan and conventional release of indapamide. Further, the dosage form should preferably display the same dissolution profile as mono-products of candesartan and indapamide. Still further, the dosage form should provide a satisfactory shelf-life/storage stability and patient compliance

This object has been achieved by the subject-matter of the patent claims.

It has been surprisingly found that solid pharmaceutical dosage forms for oral administration comprising candesartan cilexetil and indapamide provide several advantages.

First of all, coadministration of candesartan and indapamide has therapeutic advantages. Combination therapy of candesartan with indapamide shows synergistic therapeutic efficacy in the treatment of hypertension. Fixed-dose combinations of candesartan and indapamide allow for reducing administered dosages and/or administration frequencies, especially upon oral administration. The advantages for the patients are improved convenience, better assurance of compliance, reduction of severity and frequency of side effects, because such combinations maintain substantially constant blood levels and avoid the fluctuations associated with conventional immediate formulations that are administered more than once a day.

Further, it has been surprisingly found that advantageous pharmaceutical dosage forms can be prepared when indapamide is directly added into the tablet, i.e. present in an extragranular phase. Advantageous properties normally attributed to a wet granulation process, such as homogeneity of indapamide, can be combined with the advantages of a direct compression process, such as avoiding of exposing indapamide to granulating liquids which may be the primary cause of instability and moreover require an additional drying step to remove the granulation liquid in order to give the final dosage form. Thus, pharmaceutical dosage forms can be provided with a good uniformity and stability of indapamide, even though the technological process of its preparation does not include granulation of indapamide.

Still further, it has been found that selecting appropriate excipients enables adding indapamide directly into the tablet blend. In particular, it has been surprisingly found that a high amount of extragranular diluent such as microcrystalline cellulose provides satisfactory content uniformity and stability of indapamide.

Furthermore, the selected excipients and their composition also allow relatively low compression forces for preparing solid pharmaceutical forms, which has a positive effect on the stability of candesartan. Candesartan is stable against temperature, moisture and light when it is present alone in the solid state. However, when prepared into tablets by incorporating other ingredients, it has been observed that lowering of the content of the active ingredient is apt to be enhanced in the course of time due to deformation of crystals caused by pressure, abrasion and heat, applied during granulation or molding under elevated pressure during preparation.

Unless expressly stated otherwise, all percentages are expressed as weight percent. Unless expressly stated otherwise, all percentages are based on the total weight of the pharmaceutical dosage form according to the invention.

For the purpose of the specification, unless expressly stated otherwise, *"essentially consisting of"* or *"essentially the total amount"* means at least 95 wt.-%, preferably at least 98 wt.-%, more preferably at least 99 wt.-%.

Unless expressly stated otherwise, all references to Ph. Eur., ASTM, EN ISO and the like refer to the official version that is valid on September 1, 2022.

A first aspect of the invention relates to a solid pharmaceutical dosage form for oral administration comprising (i) an intragranular phase comprising candesartan cilexetil; and (ii) an extragranular phase comprising indapamide. The invention further relates to a process for preparing such pharmaceutical dosage form.

The pharmaceutical dosage form according to the invention preferably comprises a solid mixture comprising granules, i.e. the intragranular phase and a powder, i.e. the extragranular phase. Preferably, the pharmaceutical dosage form according to the invention essentially consists of the intragranular phase and the extragranular phase.

The term *"candesartan cilexetil"* as used herein designates the cyclohexyl 1-hydroxyethyl carbonate (cilexetil) ester of candesartan in its any known form or any polymorph form known from the state of the art. Candesartan cilexetil used in the pharmaceutical dosage form according to the invention may be prepared according to any manufacturing process known from the state of the art.

Preferably, besides candesartan cilexetil no other form of candesartan is present.

Preferably, essentially the total amount of the candesartan cilexetil that is contained in the dosage form is present in the intragranular phase.

Preferably, the dosage form according to the invention provides conventional release of candesartan.

The term *"conventional release"* (formerly referred to as *"immediate release"*) refers to fast dissolution characteristics such that the active ingredient candesartan cilexetil readily dissolves in a physiological aqueous medium. Preferably, *"conventional release"* is synonymous to *"immediate release".* Preferably, *"conventional release"* means that in an *in vitro* dissolution test according to Ph. Eur., basket method (40 mesh), 100 rpm, in 900 mL pH 6.8 phosphate buffer and at 37.0°C±0.5°C after 45 minutes at least 75 wt.-% of the active ingredient have been dissolved (released), relative to the total amount of this active ingredient that was originally contained in the dosage form. This definition of *"conventional release"* is in accordance with e.g. Ph. Eur. 5.17.1. *"Recommendations on dissolution testing".*

Preferably, the weight content of candesartan cilexetil is at least 2.0 wt.-%, preferably at least 4.0 wt.-%, more preferably at least 6.0 wt.-%, still more preferably at least 8.0 wt.-%, yet more preferably at least 10 wt.-%, and even more preferably at least 12 wt.-%, relative to the total weight of the dosage form.

Preferably, the weight content of candesartan cilexetil is at most 30 wt.-%, preferably at most 25 wt.-%, more preferably at most 20 wt.-%, still more preferably at most 16 wt.-%, yet more preferably at most 14 wt.-%, even more prefer-ably at most 12 wt.-%, most preferably at most 10 wt.-%, and in particular at most 8.0 wt.-%, relative to the total weight of the dosage form.

Preferably, the weight content of candesartan cilexetil is within the range of 21±16 wt.-%, preferably 19±14 wt.-%, more preferably 17±12 wt.-%, still more preferably 15±10 wt.-%, yet more preferably 13±8.0 wt.-%, even more preferably 11±6.0 wt.-%, most preferably 9.0±4.0 wt.-%, and in particular 6.0±2.0 wt.-%, or 12±2.0 wt.-%, relative to the total weight of the dosage form.

In preferred embodiments, the dose of candesartan cilexetil is at least 2.0 mg, preferably at least 4.0 mg, more preferably at least 6.0 mg, still more preferably at least 8.0 mg, yet more preferably at least 10 mg, even more preferably at least 12 mg, most preferably at least 14 mg, and in particular at least 16 mg.

In preferred embodiments, the dose of candesartan cilexetil is at most 30 mg, preferably at most 25 mg, more preferably at most 20 mg, still more preferably at most 16 mg, yet more preferably at most 14 mg, even more preferably at most 12 mg, most preferably at most 10 mg, and in particular at most 8.0 mg.

In preferred embodiments, wherein the dose of candesartan cilexetil is within the range of 21±16 mg, preferably 19±14 mg, more preferably 17±12 mg, still more preferably 15±10 mg, yet more preferably 13±8.0 mg, even more preferably 11±6.0 mg, most preferably 9.0±4.0 mg, and in particular 8.0±2.0 mg, or 16±2.0 mg.

Preferably, the intragranular phase is prepared by wet granulation; preferably aqueous wet granulation; more preferably wherein the candesartan cilexetil is dissolved in the granulation liquid.

The term *"indapamide"* as used herein designates indapamide in its any known form or any polymorph form known from the state of the art. Indapamide used in the pharmaceutical dosage form according to the invention may be prepared according to any manufacturing process known from the state of the art.

Preferably, the indapamide is present in the non-salt form; preferably besides the non-salt form no other form of indapamide is present.

Preferably, essentially the total amount of the indapamide that is contained in the dosage form is present in the extragranular phase.

Preferably, the dosage form according to the invention provides conventional release of indapamide.

Preferably, the weight content of indapamide is at least 1.5 wt.-%, preferably at least 1.6 wt.-%, more preferably at least 1.7 wt.-%, still more preferably at least 1.8 wt.-%, yet more preferably at least 1.9 wt.-%, and even more preferably at least 2.0 wt.-%, relative to the total weight of the dosage form.

Preferably, the weight content of indapamide is at most 8.0 wt.-%, preferably at most 7.0 wt.-%, more preferably at most 6.0 wt.-%, still more preferably at most 5.0 wt.-%, yet more preferably at most 4.0 wt.-%, even more preferably at most 3.0 wt.-%, most preferably at most 2.5 wt.-%, and in particular at most 2.0 wt.-%, relative to the total weight of the dosage form.

Preferably, the weight content of indapamide is within the range of 4.0±3.5 wt.-%, preferably 3.5±3.0 wt.-%, more preferably 3.0±2.5 wt.-%, still more preferably 2.8±2.3 wt.-%, yet more preferably 2.6±2.0 wt.-%, even more preferably 2.4±1.5 wt.-%, most preferably 2.2±1.0 wt.-%, and in particular 2.0±0.5 wt.-%, relative to the total weight of the dosage form.

In preferred embodiments, the dose of indapamide is at least 0.4 mg, preferably at least 0.7 mg, more preferably at least 1.0 mg, still more preferably at least 1.3 mg, yet more preferably at least 1.6 mg, even more preferably at least 1.9 mg, most preferably at least 2.2 mg, and in particular at least 2.5 mg.

In preferred embodiments, the dose of indapamide is at most 20 mg, preferably at most 10 mg, more preferably at most 5.0 mg, still more preferably at most 4.5 mg, yet more preferably at most 4.0 mg, even more preferably at most 3.5 mg, most preferably at most 3.0 mg, and in particular at most 2.6 mg.

In preferred embodiments, the dose of indapamide is within the range of 6.0±5.0 mg, preferably 5.5±4.0 mg, more preferably 5.0±3.5 mg, still more preferably 4.5±3.0 mg, yet more preferably 4.0±2.0 mg, even more preferably 3.5±1.5 mg, most preferably 3.0±1.0 mg, and in particular 2.5±0.5 mg.

Preferably, the relative weight ratio of the indapamide to the candesartan cilexetil is within the range of from 3.0:1.0 to 1.0:15, preferably from 2.0:1.0 to 1.0:10, more preferably from 1.0:1.0 to 1.0:9.0, still more preferably from 1.0:2.0 to 1.0:8.0, and yet more preferably from 1.0:3.0 to 1.0:7.0.

Preferably, the content of candesartan cilexetil is greater than the content of indapamide.

In preferred embodiments, the relative weight ratio of the extragranular phase to the intragranular phase is within the range of from 1.0:1.0 to 1.0:2.1, preferably from 1.0:1.0 to 1.0:1.9, more preferably from 1.0:1.0 to 1.0:1.7, still more preferably from 1.0:1.0 to 1.0:1.5, and yet more preferably from 1.0:1.0 to 1.0:1.3.

In preferred embodiments of the dosage form, the weight content of the intragranular phase relative to the total weight of the dosage form is at least 35 wt.-%, preferably at least 40 wt.-%, more preferably at least 45 wt.-%, still more preferably at least 50 wt.-%, yet more preferably at least 53 wt.-%, even more preferably at least 55 wt.-%, most preferably at least 57 wt.-%, and in particular at least 59 wt.-%.

In preferred embodiments of the dosage form, the weight content of the intragranular phase relative to the total weight of the dosage form is at most 85 wt.-%, preferably at most 80 wt.-%, more preferably at most 75 wt.-%, still more preferably at most 70 wt.-%, yet more preferably at most 65 wt.-%, even more preferably at most 60 wt.-%, most preferably at most 57 wt.-%, and in particular at most 55 wt.-%.

In preferred embodiments of the dosage form, the weight content of the intragranular phase relative to the total weight of the dosage form is within the range of 56±30 wt.-%, preferably 56±25 wt.-%, more preferably 56±20 wt.-%, still more preferably 56±15 wt.-%, yet more preferably 56±10 wt.-%, even more preferably 56±8.0 wt.-%, most preferably 56±6.0 wt.-%, and in particular 56±4.0 wt.-%.

In preferred embodiments of the dosage form, the weight content of the extragranular phase relative to the total weight of the dosage form is at least 15 wt.-%, preferably at least 20 wt.-%, more preferably at least 25 wt.-%, still more preferably at least 30 wt.-%, yet more preferably at least 35 wt.-%, even more preferably at least 40 wt.-%, most preferably at least 42 wt.-%, and in particular at least 45 wt.-%.

In preferred embodiments of the dosage form, the weight content of the extragranular phase relative to the total weight of the dosage form is at most 75 wt.-%, preferably at most 70 wt.-%, more preferably at most 65 wt.-%, still more preferably at most 60 wt.-%, yet more preferably at most 55 wt.-%, even more preferably at most 50 wt.-%, most preferably at most 47 wt.-%, and in particular at most 45 wt.-%.

In preferred embodiments of the dosage form, the weight content of the extragranular phase relative to the total weight of the dosage form is within the range of 44±30 wt.-%, preferably 44±25 wt.-%, more preferably 44±20 wt.-%, still more preferably 44±15 wt.-%, yet more preferably 44±10 wt.-%, even more preferably 44±8.0 wt.-%, most preferably 44±6.0 wt.-%, and in particular 44±4.0 wt.-%.

Preferably, besides candesartan cilexetil and indapamide no other pharmacologically active ingredients are present.

In preferred embodiments, the dosage form according to the invention comprises one or more excipients independently of one another selected from binders, lubricants, diluents, disintegrants, and glidants.

Preferably, the intragranular phase comprises at least one intragranular binder; preferably selected from the group consisting of
- povidone (polyvinylpyrrolidone) or copovidone (vinylpyrrolidone-vinyl acetate copolymer);
- cellulose derivatives, cellulose esters or cellulose ethers; preferably hydroxymethyl cellulose, hydroxyethyl cellulose, hydroxypropyl cellulose, low substituted hydroxypropyl cellulose or hydroxypropylmethyl cellulose;
- polyvinyl alcohol;
- starch or starch derivatives; preferably potato starch, rice starch, α-starch, or pregelatinized starch;
- mono- or polysaccharides; preferably dextrin, gum arabic, or pullulan;
- poly(meth)acrylates; and
- mixtures thereof;
preferably cellulose derivatives, cellulose esters or cellulose ethers, more preferably hydroxypropyl cellulose.

In preferred embodiments, the weight content of the at least one intragranular binder is at least 0.2 wt.-%, preferably at least 0.6 wt.-%, more preferably at least 1.0 wt.-%, still more preferably at least 1.4 wt.-%, yet more preferably at least 1.8 wt.-%, even more preferably at least 2.2 wt.-%, most preferably at least 2.6 wt.-%, and in particular at least 3.0 wt.-%, relative to the total weight of the dosage form.

In preferred embodiments, the weight content of the at least one intragranular binder is at most 30 wt.-%, preferably at most 20 wt.-%, more preferably at most 15 wt.-%, still more preferably at most 10 wt.-%, yet more preferably at most 7.5 wt.-%, even more preferably at most 5.0 wt.-%, most preferably at most 4.0 wt.-%, and in particular at most 3.5 wt.-%, relative to the total weight of the dosage form.

In preferred embodiments, the weight content of the at least one intragranular binder is within the range of 30±28 wt.-%, preferably 25±23 wt.-%, more preferably 20±18 wt.-%, still more preferably 15±13 wt.-%, yet more preferably 10±8.0 wt.-%, even more preferably 7.0±5.0 wt.-%, most preferably 5.0±3.0 wt.-%, and in particular 3.0±1.5 wt.-%, relative to the total weight of the dosage form.

Preferably, the intragranular phase comprises at least one intragranular lubricant; preferably selected from the group consisting of
- C₁₂₋₂₀-fatty acids and C₁₂₋₂₀-fatty acid esters, preferably selected from glyceryl monostearate, glyceryl tribehenate, and glyceryl dibehenate;
- metal salts of C₁₂₋₂₀-fatty acids and derivatives thereof; preferably selected from calcium stearate, aluminum stearate, zinc stearate, magnesium palmitate, magnesium oleate, and sodium stearyl fumarate;
- polyalkylene glycols and polyalkylene oxides, preferably polyethylene glycols (PEG), more preferably PEG 8000, PEG 4600, PEG 4000, PEG 3350, PEG 3000, PEG 2000, PEG 1540, PEG 1450, or PEG 1000;
- hydrogenated oils, preferably hydrogenated vegetable oil or hydrogenated castor oil;
- waxes, preferably meads wax or spermaceti;
- boric acid; and
- mixtures thereof;
preferably polyalkylene glycols, more preferably polyethylene glycol, still more preferably PEG 8000.

In preferred embodiments, the weight content of the at least one intragranular lubricant is at least 1.3 wt.-%, preferably at least 1.4 wt.-%, more preferably at least 1.5 wt.-%, still more preferably at least 1.6 wt.-%, yet more preferably at least 1.7 wt.-%, even more preferably at least 1.8 wt.-%, most preferably at least 1.9 wt.-%, and in particular at least 2.0 wt.-%, relative to the total weight of the dosage form.

In preferred embodiments, the weight content of the at least one intragranular lubricant is at most 30 wt.-%, preferably at most 20 wt.-%, more preferably at most 15 wt.-%, still more preferably at most 10 wt.-%, yet more preferably at most 5.0 wt.-%, even more preferably at most 3.0 wt.-%, most preferably at most 2.5 wt.-%, and in particular at most 2.0 wt.-%, relative to the total weight of the dosage form.

In preferred embodiments, the weight content of the at least one intragranular lubricant is within the range of 30±28 wt.-%, preferably 25±23 wt.-%, more preferably 20±18 wt.-%, still more preferably 15±13 wt.-%, yet more preferably 10±8.0 wt.-%, even more preferably 7.0±5.0 wt.-%, most preferably 5.0±3.0 wt.-%, and in particular 2.0±1.5 wt.-%, relative to the total weight of the dosage form.

Preferably, the intragranular phase comprises at least one intragranular diluent; preferably selected from the group consisting of
- monosaccharides, disaccharides, and oligosaccharides or their derivatives; preferably selected from lactose, e.g. lactose monohydrate, anhydrous lactose, spray dried and/or granulated lactose, lactose hydrate, partially amorphous lactose, completely amorphous lactose; glucose, fructose, sucrose, dextrates, saccharose, raffinose, trehalose, isomaltose, lactitol, and dextrin;
- sugar alcohols; preferably selected from xylitol, mannitol, maltitol, isomalt, erythritol, and sorbitol;
- starch or starch derivatives; preferably wheat starch, rice starch, tapioca starch, potato starch, pregelatinized starch, or low moisture pregelatinized starch;
- silicates; preferably magnesium aluminometasilicate;
- salts of phosphoric acid; preferably calcium salts of phosphoric acid; preferably selected from calcium hydrogen phosphate anhydrous and calcium hydrogen phosphate hydrate;
- salts of carbonic acid; preferably calcium carbonate, sodium carbonate, potassium carbonate, calcium hydrogencarbonate, sodium hydrogencarbonate, or potassium hydrogencarbonate;
- salts of lactic acid; preferably calcium lactate; and
- mixtures thereof;
preferably lactose, more preferably lactose monohydrate.

In preferred embodiments, the weight content of the at least one intragranular diluent is at least 10 wt.-%, preferably at least 20 wt.-%, more preferably at least 25 wt.-%, still more preferably at least 30 wt.-%, yet more preferably at least 32 wt.-%, even more preferably at least 34 wt.-%, most preferably at least 36 wt.-%, and in particular at least 38 wt.-%, relative to the total weight of the dosage form.

In preferred embodiments, the weight content of the at least one intragranular diluent is at most 70 wt.-%, preferably at most 65 wt.-%, more preferably at most 60 wt.-%, still more preferably at most 55 wt.-%, yet more preferably at most 50 wt.-%, even more preferably at most 45 wt.-%, most preferably at most 40 wt.-%, and in particular at most 35 wt.-%, relative to the total weight of the dosage form.

In preferred embodiments, the weight content of the at least one intragranular diluent is within the range of 70±30 wt.-%, preferably 65±35 wt.-%, more preferably 60±40 wt.-%, still more preferably 55±30 wt.-%, yet more preferably 50±25 wt.-%, even more preferably 45±20 wt.-%, most preferably 40±15 wt.-%, and in particular 35±5.0 wt.-%, relative to the total weight of the dosage form.

For the purpose of the specification a diluent is considered equivalent to a filler. Thus, both excipients perform the same functions, namely to increase the weight and improve the uniformity of the contents.

Preferably, the intragranular phase comprises at least one intragranular disintegrant; preferably selected from the group consisting of
- crospovidone;
- starch and starch derivatives; preferably corn starch (maize starch), native starch, pregelatinized starch, sodium starch glycollate, hydroxypropyl starch, or carboxymethyl starch;
- heteroglycanes; preferably alginic acid, sodium alginate, calcium alginate, agar, or guar gum;
- glucosamines; preferably chitosan;
- polacrilin potassium or docusate sodium; and
- mixtures thereof;
preferably starch, more preferably corn starch.

In preferred embodiments, the weight content of the at least one intragranular disintegrant is at least 1.0 wt.-%, preferably at least 2.0 wt.-%, more preferably at least 3.0 wt.-%, still more preferably at least 4.0 wt.-%, yet more preferably at least 5.0 wt.-%, even more preferably at least 6.0 wt.-%, most preferably at least 7.0 wt.-%, and in particular at least 8.0 wt.-%, relative to the total weight of the dosage form.

In preferred embodiments, the weight content of the at least one intragranular disintegrant is at most 40 wt.-%, preferably at most 35 wt.-%, more preferably at most 30 wt.-%, still more preferably at most 25 wt.-%, yet more preferably at most 20 wt.-%, even more preferably at most 15 wt.-%, most preferably at most 12 wt.-%, and in particular at most 10 wt.-%, relative to the total weight of the dosage form.

In preferred embodiments, the weight content of the at least one intragranular disintegrant is within the range of 30±28 wt.-%, preferably 25±23 wt.-%, more preferably 20±18 wt.-%, still more preferably 17±13 wt.-%, yet more preferably 15±8.0 wt.-%, even more preferably 13±6.0 wt.-%, most preferably 11±4.0 wt.-%, and in particular 9.0±2.0 wt.-%, relative to the total weight of the dosage form.

Preferably, the extragranular phase comprises at least one extragranular diluent; preferably selected from the group consisting of
- sugar alcohols; preferably selected from xylitol, mannitol, maltitol, isomalt, erythritol, and sorbitol;
- starch or starch derivatives; preferably wheat starch, rice starch, tapioca starch, potato starch, pregelatinized starch, or low moisture pregelatinized starch;
- cellulose or cellulose derivatives; preferably powdered cellulose, microcrystalline cellulose, or silicified microcrystalline cellulose;
- silicates; preferably magnesium aluminometasilicate;
- salts of phosphoric acid; preferably calcium salts of phosphoric acid; preferably selected from calcium hydrogen phosphate anhydrous and calcium hydrogen phosphate hydrate;
- salts of carbonic acid; preferably calcium carbonate, sodium carbonate, potassium carbonate, calcium hydrogencarbonate, sodium hydrogencarbonate, or potassium hydrogen-carbonate;
- salts of lactic acid; preferably calcium lactate; and
- mixtures thereof;
preferably cellulose or cellulose derivatives, more preferably microcrystalline cellulose.

In preferred embodiments, the weight content of the at least one extragranular diluent is at least 10 wt.-%, preferably at least 20 wt.-%, more preferably at least 25 wt.-%, still more preferably at least 30 wt.-%, yet more preferably at least 32 wt.-%, even more preferably at least 36 wt.-%, most preferably at least 38 wt.-%, and in particular at least 40 wt.-%, relative to the total weight of the dosage form.

In preferred embodiments, the weight content of the at least one extragranular diluent is at most 75 wt.-%, preferably at most 70 wt.-%, more preferably at most 65 wt.-%, still more preferably at most 60 wt.-%, yet more preferably at most 55 wt.-%, even more preferably at most 50 wt.-%, most preferably at most 45 wt.-%, and in particular at most 40 wt.-%, relative to the total weight of the dosage form.

In preferred embodiments, the weight content of the at least one extragranular diluent is within the range of 70±30 wt.-%, preferably 65±35 wt.-%, more preferably 60±40 wt.-%, still more preferably 55±30 wt.-%, yet more preferably 50±25 wt.-%, even more preferably 45±20 wt.-%, most preferably 40±10 wt.-%, and in particular 35±5.0 wt.-%, relative to the total weight of the dosage form.

In preferred embodiments, the weight content of the at least one extragranular diluent is at least 60 wt.-%, preferably at least 65 wt.-%, more preferably at least 70 wt.-%, still more preferably at least 75 wt.-%, yet more preferably at least 80 wt.-%, even more preferably at least 84 wt.-%, most preferably at least 86 wt.-%, and in particular at least 88 wt.-%, relative to the total weight of the extragranular phase.

In preferred embodiments, the weight content of the at least one extragranular diluent is at most 99 wt.-%, preferably at most 98 wt.-%, more preferably at most 96 wt.-%, still more preferably at most 94 wt.-%, yet more preferably at most 92 wt.-%, even more preferably at most 90 wt.-%, most preferably at most 88 wt.-%, and in particular at most 86 wt.-%, relative to the total weight of the extragranular phase.

In preferred embodiments, wherein the weight content of the at least one extragranular diluent is within the range of 60±35 wt.-%, preferably 65±30 wt.-%, more preferably 70±20 wt.-%, still more preferably 75±15 wt.-%, yet more preferably 80±10 wt.-%, even more preferably 82±7.0 wt.-%, most preferably 84±5.0 wt.-%, and in particular 86±3.0 wt.-%, relative to the total weight of the extragranular phase.

In preferred embodiments, the at least one extragranular diluent, preferably microcrystalline cellulose, has an average particle size of at least 10 µm, preferably at least 20 µm, more preferably at least 30 µm, still more preferably at least 40 µm, and yet more preferably at least 50 µm; preferably determined in accordance with Ph. Eur., 2.9.31: Particle Size Analysis by Laser Light Diffraction.

In preferred embodiments, the at least one extragranular diluent, preferably microcrystalline cellulose, has an average particle size of at most 200 µm, preferably at most 180 µm, more preferably at most 160 µm, still more preferably at most 140 µm, yet more preferably at most 120 µm, and even more preferably at most 100 µm; preferably determined in accordance with Ph. Eur., 2.9.31: Particle Size Analysis by Laser Light Diffraction.

In preferred embodiments, the at least one extragranular diluent, preferably microcrystalline cellulose, has an average particle size within the range of 100±90 µm, preferably 100±80 µm, more preferably 100±70 µm, still more preferably 100±60 µm, yet more preferably 100±50 µm, even more preferably 100±40 µm, or 50±40 µm, most preferably 100±30 µm, or 50±30 µm, preferably determined in accordance with Ph. Eur., 2.9.31: Particle Size Analysis by Laser Light Diffraction.

Preferably, the extragranular phase comprises at least one extragranular disintegrant; preferably selected from the group consisting of
- crospovidone;
- cellulose or cellulose derivatives; methylcellulose, sodium salts of carboxymethyl cellulose, calcium salts of carboxymethyl cellulose, or cross-linked carboxymethylcellulose, e.g. croscarmellose sodium and/or croscarmellose calcium;
- heteroglycanes; preferably alginic acid, sodium alginate, calcium alginate, agar, or guar gum;
- glucosamines; preferably chitosan;
- polacrilin potassium or docusate sodium; and
- mixtures thereof;
preferably cellulose or cellulose derivatives, more preferably croscarmellose calcium.

In preferred embodiments, the weight content of the at least one extragranular disintegrant is at least 0.2 wt.-%, preferably at least 0.6 wt.-%, more preferably at least 1.0 wt.-%, still more preferably at least 1.4 wt.-%, yet more preferably at least 1.8 wt.-%, even more preferably at least 2.2 wt.-%, most preferably at least 2.6 wt.-%, and in particular at least 3.0 wt.-%, relative to the total weight of the dosage form.

In preferred embodiments, the weight content of the at least one extragranular disintegrant is at most 8.0 wt.-%, preferably at most 7.0 wt.-%, more preferably at most 6.0 wt.-%, still more preferably at most 5.0 wt.-%, yet more preferably at most 4.0 wt.-%, even more preferably at most 3.0 wt.-%, most preferably at most 2.5 wt.-%, and in particular at most 2.0 wt.-%, relative to the total weight of the dosage form.

In preferred embodiments, the weight content of the at least one extragranular disintegrant is within the range of 10±9.0 wt.-%, preferably 8.0±7.0 wt.-%, more preferably 7.0±6.0 wt.-%, still more preferably 6.0±5.0 wt.-%, yet more preferably 5.0±4.0 wt.-%, even more preferably 4.0±3.0 wt.-%, most preferably 3.0±2.0 wt.-%, and in particular 2.5±1.0 wt.-%, relative to the total weight of the dosage form.

Preferably, the extragranular phase comprises at least one extragranular glidant; preferably selected from the group consisting of colloidal silica, talc, magnesium trisilicate, and mixtures thereof; preferably colloidal silica, more preferably colloidal anhydrous silica.

In preferred embodiments, the weight content of the at least one extragranular glidant is within the range of 5.0±4.5 wt.-%, preferably 2.0±1.5 wt.-%, more preferably 1.5±1.0 wt.-%, still more preferably 1.0±0.8 wt.-%, yet more preferably 0.8±0.6 wt.-%, even more preferably 0.7±0.5 wt.-%, most preferably 0.6±0.4 wt.-%, and in particular 0.5±0.4 wt.-%, relative to the total weight of the dosage form.

Preferably, the extragranular phase comprises at least one extragranular lubricant; preferably selected from the group consisting of
- C₁₂₋₂₀-fatty acids and C₁₂₋₂₀-fatty acid esters, preferably selected from glyceryl monostearate, glyceryl tribehenate, and glyceryl dibehenate;
- metal salts of C₁₂₋₂₀-fatty acids and derivatives thereof; preferably selected from magnesium stearate, calcium stearate, aluminum stearate, zinc stearate, magnesium palmitate, magnesium oleate, and sodium stearyl fumarate;
- hydrogenated oils, preferably hydrogenated vegetable oil or hydrogenated castor oil;
- waxes, preferably meads wax or spermaceti;
- boric acid; and
- mixtures thereof;
preferably magnesium stearate.

In preferred embodiments, the weight content of the at least one extragranular lubricant is within the range 5.0±4.5 wt.-%, preferably 2.0±1.5 wt.-%, more preferably 1.5±1.0 wt.-%, still more preferably 1,0±0.8 wt.-%, yet more preferably 0.8±0.6 wt.-%, even more preferably 0.7±0.5 wt.-%, most preferably 0.6±0.5 wt.-%, and in particular 0.6±0.4 wt.-%, relative to the total weight of the dosage form.

Preferably, the pharmaceutical dosage form according to the invention comprises at least one coloring agent; preferably selected from dyes and pigments such as iron oxide red, yellow, brown or black, titanium dioxide and combinations thereof; preferably iron oxide red.

In preferred embodiments, the pharmaceutical dosage form according to the invention comprises or essentially consists of
(i) the intragranular phase comprising
   - candesartan cilexetil;
   - optionally, at least one intragranular binder, preferably hydroxypropyl cellulose, more preferably at a weight content within the range of 3.0±2.0 wt.-%;
   - optionally, at least one intragranular lubricant, preferably polyethylene glycol, more preferably at a weight content within the range of 2.0±1.5 wt.-%;
   - at least one intragranular diluent, preferably lactose monohydrate, more preferably at a weight content within the range of 35±10 wt.-%; and
   - at least one intragranular disintegrant, preferably corn starch, more preferably at a weight content within the range of 9.0±4.0 wt.-%; and
(ii) the extragranular phase comprising
   - indapamide;
   - at least one extragranular diluent, preferably microcrystalline cellulose, more preferably at a weight content within the range of 35±10 wt.-%;
   - optionally, at least one extragranular disintegrant, preferably carmellose calcium, more preferably at a weight content within the range of 2.5±2.0 wt.-%;
   - optionally, at least one extragranular glidant, preferably colloidal anhydrous silica, more preferably at a weight content within the range of 0.5±0.2 wt.-%; and
   - optionally, at least one extragranular lubricant, preferably magnesium stearate, more preferably at a weight content within the range of 0.6±0.2 wt.-%;
in each case relative to the total weight of the dosage form.

Preferably, the dosage form according to the invention is compacted or compressed.

Preferably, the dosage form according to the invention is selected from the group consisting of tablets, pills, powders, lozenges, sachets, soft gelatin capsules, hard gelatin capsules, and suppositories; preferably tablets.

Preferably, the dosage form according to the invention is coated; preferably with a water-soluble coating material.

In preferred embodiments, the overall weight of the dosage form is within the range of 130±50 mg, preferably 130±40 mg, more preferably 130±30 mg, still more preferably 130±25 mg, yet more preferably 130±20 mg, even more preferably 130±15 mg, most prefer-ably 130±10 mg, and in particular 130±5.0 mg.

Preferably, the acceptance value of candesartan cilexetil is at most 8.0, preferably at most 6.0, more preferably at most 5.0, still more preferably at most 4.5, yet more preferably at most 4.0, even more preferably at most 3.5, most preferably at most 3.0, and in particular at most 2.5; preferably determined according to the content uniformity test in accordance with Ph. Eur.

Preferably, the acceptance value of candesartan cilexetil is within the range of 2.0±0.5, or 2.5±1.0, or 2.5±0.5, or 3.0±1.5, or 3.0±1.0, or 3.0±0.5, or 3.5±2.0, or 3.5±1.5, or 3.5±1.0, or 3.5±0.5, or 4.0±2.5, or 4.0±2.0, or 4.0±1.5, or 4.0±1.0, or 4.0±0.5, or 4.5±3.0, or 4.5±2.5, or 4.5±2.0, or 4.5±1.5, or 4.5±1.0, or 4.5±0.5, or 5.0±3.5, or 5.0±3.0, or 5.0±2.5, or 5.0±2.0, or 5.0±1.5, or 5.0±1.0, or 5.0±0.5; preferably determined according to the content uniformity test in accordance with Ph. Eur.

The term *"content uniformity test"* refers to an assay of the individual content of active ingredient(s) in a number of dosage units to determine whether the individual content is within the limits set. *"Dosage units"* are preferably defined as dosage forms containing a single dose or a part of a dose of active ingredient in each unit. To ensure the consistency of dosage units, each unit in a batch preferably has a content of the active pharmaceutical ingredient within a narrow range around the specified dose. For the purpose of the specification the term *"uniformity of a dosage unit"* is defined as the degree of uniformity in the amount of the active ingredient dosage units.

The term *"acceptance value"* (AV) refers to a criterion on content uniformity. The acceptance value preferably is a function of deviation of the mean from the specified dose by more than 1.5% and the standard deviation of the results. Preferably, the requirements for dosage uniformity are met if the acceptance value of the first 10 dosage units is less than or equal to 15.

Preferably, the acceptance value of indapamide is at most 8.0, preferably at most 6.0, more preferably at most 5.0, still more preferably at most 4.5, yet more preferably at most 4.0, even more preferably at most 3.5, most preferably at most 3.0, and in particular at most 2.5; preferably determined according to the content uniformity test in accordance with Ph. Eur.

Preferably, the acceptance value of indapamide is within the range of 2.0±0.5, or 2.5±1.0, or 2.5±0.5, or 3.0±1.5, or 3.0±1.0, or 3.0±0.5, or 3.5±2.0, or 3.5±1.5, or 3.5±1.0, or 3.5±0.5, or 4.0±2.5, or 4.0±2.0, or 4.0±1.5, or 4.0±1.0, or 4.0±0.5, or 4.5±3.0, or 4.5±2.5, or 4.5±2.0, or 4.5±1.5, or 4.5±1.0, or 4.5±0.5, or 5.0±3.5, or 5.0±3.0, or 5.0±2.5, or 5.0±2.0, or 5.0±1.5, or 5.0±1.0, or 5.0±0.5; preferably determined according to the content uniformity test in accordance with Ph. Eur.

Preferably, the content of indapamide impurity A (2-methyl-1-nitroso-2,3-dihydroindole) is at most 10 ppm, preferably at most 6.0 ppm, more preferably at most 4.0 ppm, still more preferably at most 3.5 ppm, yet more preferably at most 3.0 ppm, even more preferably at most 2.5 ppm, most preferably at most 2.0 ppm, and in particular at most 1.5 ppm; preferably after storage of the dosage form according to the invention in aluminum - PVDC/PVC 250/90 blisters for 3 months under conditions of 40°C/75%.

Another aspect of the invention relates to the dosage form according to the invention for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supra-ventricular and ventricular arrhythmias, atrial fibrillation, atrial flutter, detrimental vascular re-modeling, myocardial infarction and its sequelae, atherosclerosis, angina (whether unstable or stable), renal insufficiency (diabetic and non-diabetic), angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.

Still another aspect of the invention relates to a process for the preparation of a dosage form according to the invention.

The process according to the invention preferably comprises the steps of:
(a) providing a granulation liquid comprising
   - candesartan cilexetil;
   - one or more solvents, preferably water; and
   - optionally, one or more excipients, preferably at least one intragranular binder as defined above and/or at least one intragranular lubricant as defined above;
(b) providing a granulation mixture comprising one or more excipients, preferably at least one intragranular diluent as defined above and/or at least one intragranular disintegrant as defined above;
(c) wet granulating the granulation mixture provided in step (b) with the granulation liquid provided in step (a) thereby obtaining an intragranular phase;
(d) mixing the intragranular phase obtained in step (c) with indapamide and optionally one or more excipients, preferably at least one extragranular diluent as defined above, at least one extragranular disintegrant as defined above, at least one extragranular glidant as defined above, and/or at least one extragranular lubricant as defined above, thereby obtaining a compression mixture which comprises the intragranular phase obtained in step (c) and an extragranular phase comprising the indapamide and the optionally present one or more excipients, preferably at least one extragranular diluent, at least one extragranular disintegrant, at least one extragranular glidant, and/or at least one extragranular lubricant;
(e) optionally, compressing the compression mixture provided in step (d) by means of a tablet press thereby obtaining a tablet.

Preferably, in step (c) of the process according to the invention the wet granulation is performed by means of a granulating device; preferably a high shear mixer or a fluid bed dryer.

In preferred embodiments, step (c) of the process according to the invention involves drying and/or sieving of the intragranular phase thereby obtaining a dried and/or sieved intragranular phase.

Preferably, in step (d) of the process according to the invention the mixing is performed by means of a mixing device; preferably a container mixer.

Another aspect of the invention relates to a pharmaceutical dosage form obtainable by the process according to the invention.

Particularly preferred embodiments of the invention are compiled as clauses 1 to 76 hereinafter:
Clause 1: A solid pharmaceutical dosage form for oral administration comprising
   (i) an intragranular phase comprising candesartan cilexetil; and
   (ii) an extragranular phase comprising indapamide.
Clause 2: The dosage form according to clause 1, which provides conventional release of candesartan.
Clause 3: The dosage form according to clause 1 or 2, wherein the weight content of candesartan cilexetil is at least 2.0 wt.-%, preferably at least 4.0 wt.-%, more preferably at least 6.0 wt.-%, still more preferably at least 8.0 wt.-%, yet more preferably at least 10 wt.-%, and even more preferably at least 12 wt.-%, relative to the total weight of the dosage form.
Clause 4: The dosage form according to any of the preceding clauses, wherein the weight content of candesartan cilexetil is at most 30 wt.-%, preferably at most 25 wt.-%, more preferably at most 20 wt.-%, still more preferably at most 16 wt.-%, yet more preferably at most 14 wt.-%, even more prefer-ably at most 12 wt.-%, most preferably at most 10 wt.-%, and in particular at most 8.0 wt.-%, relative to the total weight of the dosage form.
Clause 5: The dosage form according to any of the preceding clauses, wherein the weight content of candesartan cilexetil is within the range of 21±16 wt.-%, preferably 19±14 wt.-%, more preferably 17±12 wt.-%, still more preferably 15±10 wt.-%, yet more preferably 13±8.0 wt.-%, even more preferably 11±6.0 wt.-%, most preferably 9.0±4.0 wt.-%, and in particular 6.0±2.0 wt.-%, or 12±2.0 wt.-%, relative to the total weight of the dosage form.
Clause 6: The dosage form according to any of the preceding clauses, wherein the dose of candesartan cilexetil is at least 2.0 mg, preferably at least 4.0 mg, more preferably at least 6.0 mg, still more preferably at least 8.0 mg, yet more preferably at least 10 mg, even more preferably at least 12 mg, most preferably at least 14 mg, and in particular at least 16 mg.
Clause 7: The dosage form according to any of the preceding clauses, wherein the dose of candesartan cilexetil is at most 30 mg, preferably at most 25 mg, more preferably at most 20 mg, still more preferably at most 16 mg, yet more preferably at most 14 mg, even more preferably at most 12 mg, most preferably at most 10 mg, and in particular at most 8.0 mg.
Clause 8: The dosage form according to any of the preceding clauses, wherein the dose of candesartan cilexetil is within the range of 21±16 mg, preferably 19±14 mg, more preferably 17±12 mg, still more preferably 15±10 mg, yet more preferably 13±8.0 mg, even more preferably 11±6.0 mg, most preferably 9.0±4.0 mg, and in particular 8.0±2.0 mg, or 16±2.0 mg.
Clause 9: The dosage form according to clause 1 or 2, wherein besides candesartan cilexetil no other form of candesartan is present.
Clause 10: The dosage form according to any of the preceding clauses, wherein essentially the total amount of the candesartan cilexetil that is contained in the dosage form is present in the intragranular phase.
Clause 11: The dosage form according to any of the preceding clauses, wherein the intragranular phase is prepared by wet granulation; preferably aqueous wet granulation; more preferably wherein the candesartan cilexetil is dissolved in the granulation liquid.
Clause 12: The dosage form according to any of the preceding clauses, which provides conventional release of indapamide.
Clause 13: The dosage form according to any of the preceding clauses, wherein the weight content of indapamide is at least 1.5 wt.-%, preferably at least 1.6 wt.-%, more preferably at least 1.7 wt.-%, still more preferably at least 1.8 wt.-%, yet more preferably at least 1.9 wt.-%, and even more preferably at least 2.0 wt.-%, relative to the total weight of the dosage form.
Clause 14: The dosage form according to any of the preceding clauses, wherein the weight content of indapamide is at most 8.0 wt.-%, preferably at most 7.0 wt.-%, more preferably at most 6.0 wt.-%, still more preferably at most 5.0 wt.-%, yet more preferably at most 4.0 wt.-%, even more preferably at most 3.0 wt.-%, most preferably at most 2.5 wt.-%, and in particular at most 2.0 wt.-%, relative to the total weight of the dosage form.
Clause 15: The dosage form according to any of the preceding clauses, wherein the weight content of indapamide is within the range of 4.0±3.5 wt.-%, preferably 3.5±3.0 wt.-%, more preferably 3.0±2.5 wt.-%, still more preferably 2.8±2.3 wt.-%, yet more preferably 2.6±2.0 wt.-%, even more preferably 2.4±1.5 wt.-%, most preferably 2.2±1.0 wt.-%, and in particular 2.0±0.5 wt.-%, relative to the total weight of the dosage form.
Clause 16: The dosage form according to any of the preceding clauses, wherein the dose of indapamide is at least 0.4 mg, preferably at least 0.7 mg, more preferably at least 1.0 mg, still more preferably at least 1.3 mg, yet more preferably at least 1.6 mg, even more preferably at least 1.9 mg, most preferably at least 2.2 mg, and in particular at least 2.5 mg.
Clause 17: The dosage form according to any of the preceding clauses, wherein the dose of indapamide is at most 20 mg, preferably at most 10 mg, more preferably at most 5.0 mg, still more preferably at most 4.5 mg, yet more preferably at most 4.0 mg, even more preferably at most 3.5 mg, most preferably at most 3.0 mg, and in particular at most 2.6 mg.
Clause 18: The dosage form according to any of the preceding clauses, wherein the dose of indapamide is within the range of 6.0±5.0 mg, preferably 5.5±4.0 mg, more preferably 5.0±3.5 mg, still more preferably 4.5±3.0 mg, yet more preferably 4.0±2.0 mg, even more preferably 3.5±1.5 mg, most preferably 3.0±1.0 mg, and in particular 2.5±0.5 mg.
Clause 19: The dosage form according to any of the preceding clauses, wherein the indapamide is present in the non-salt form; preferably wherein besides the non-salt form no other form of indapamide is present.
Clause 20: The dosage form according to any of the preceding clauses, wherein essentially the total amount of the indapamide that is contained in the dosage form is present in the extragranular phase.
Clause 21: The dosage form according to any of the preceding clauses, wherein the relative weight ratio of the indapamide to the candesartan cilexetil is within the range of from 3.0:1.0 to 1.0:15, preferably from 2.0:1.0 to 1.0:10, more preferably from 1.0:1.0 to 1.0:9.0, still more preferably from 1.0:2.0 to 1.0:8.0, and yet more preferably from 1.0:3.0 to 1.0:7.0.
Clause 22: The dosage form according to any of the preceding clauses, wherein the content of candesartan cilexetil is greater than the content of indapamide.
Clause 23: The dosage form according to any of the preceding clauses, wherein the relative weight ratio of the extragranular phase to the intragranular phase is within the range of from 1.0:1.0 to 1.0:2.1, preferably from 1.0:1.0 to 1.0:1.9, more preferably from 1.0:1.0 to 1.0:1.7, still more preferably from 1.0:1.0 to 1.0:1.5, and yet more preferably from 1.0:1.0 to 1.0:1.3.
Clause 24: The dosage form according to any of the preceding clauses, wherein besides candesartan cilexetil and indapamide no other pharmacologically active ingredients are present.
Clause 25: The dosage form according to any of the preceding clauses, which comprises one or more excipients independently of one another selected from binders, lubricants, diluents, disintegrants, and glidants.
Clause 26: The dosage form according to any of the preceding clauses, wherein the intragranular phase comprises at least one intragranular binder; preferably selected from the group consisting of
   - povidone (polyvinylpyrrolidone) or copovidone (vinylpyrrolidone-vinyl acetate copolymer);
   - cellulose derivatives, cellulose esters or cellulose ethers; preferably hydroxymethyl cellulose, hydroxyethyl cellulose, hydroxypropyl cellulose, low substituted hydroxypropyl cellulose or hydroxypropylmethyl cellulose;
   - polyvinyl alcohol;
   - starch or starch derivatives; preferably potato starch, rice starch, α-starch, or pregelatinized starch;
   - mono- or polysaccharides; preferably dextrin, gum arabic, or pullulan;
   - poly(meth)acrylates; and
   - mixtures thereof; preferably cellulose derivatives, cellulose esters or cellulose ethers, more preferably hydroxypropyl cellulose.
Clause 27: The dosage form according to clause 26, wherein the weight content of the at least one intragranular binder is at least 0.2 wt.-%, preferably at least 0.6 wt.-%, more preferably at least 1.0 wt.-%, still more preferably at least 1.4 wt.-%, yet more preferably at least 1.8 wt.-%, even more preferably at least 2.2 wt.-%, most preferably at least 2.6 wt.-%, and in particular at least 3.0 wt.-%, relative to the total weight of the dosage form.
Clause 28: The dosage form according to clause 26 or 27, wherein the weight content of the at least one intragranular binder is at most 30 wt.-%, preferably at most 20 wt.-%, more preferably at most 15 wt.-%, still more preferably at most 10 wt.-%, yet more preferably at most 7.5 wt.-%, even more preferably at most 5.0 wt.-%, most preferably at most 4.0 wt.-%, and in particular at most 3.5 wt.-%, relative to the total weight of the dosage form.
Clause 29: The dosage form according to any of clauses 26 to 28, wherein the weight content of the at least one intragranular binder is within the range of 30±28 wt.-%, preferably 25±23 wt.-%, more preferably 20±18 wt.-%, still more preferably 15±13 wt.-%, yet more preferably 10±8.0 wt.-%, even more preferably 7.0±5.0 wt.-%, most preferably 5.0±3.0 wt.-%, and in particular 3.0±1.5 wt.-%, relative to the total weight of the dosage form.
Clause 30: The dosage form according to any of the preceding clauses, wherein the intragranular phase comprises at least one intragranular lubricant; preferably selected from the group consisting of
   - C₁₂₋₂₀-fatty acids and C₁₂₋₂₀-fatty acid esters, preferably selected from glyceryl monostearate, glyceryl tribehenate, and glyceryl dibehenate;
   - metal salts of C₁₂₋₂₀-fatty acids and derivatives thereof; preferably selected from calcium stearate, aluminum stearate, zinc stearate, magnesium palmitate, magnesium oleate, and sodium stearyl fumarate;
   - polyalkylene glycols and polyalkylene oxides, preferably polyethylene glycols (PEG), more preferably PEG 8000, PEG 4600, PEG 4000, PEG 3350, PEG 3000, PEG 2000, PEG 1540, PEG 1450, or PEG 1000;
   - hydrogenated oils, preferably hydrogenated vegetable oil or hydrogenated castor oil;
   - waxes, preferably meads wax or spermaceti;
   - boric acid; and
   - mixtures thereof; preferably polyalkylene glycols, more preferably polyethylene glycol, still more preferably PEG 8000.
Clause 31: The dosage form according to clause 30, wherein the weight content of the at least one intragranular lubricant is at least 1.3 wt.-%, preferably at least 1.4 wt.-%, more preferably at least 1.5 wt.-%, still more preferably at least 1.6 wt.-%, yet more preferably at least 1.7 wt.-%, even more preferably at least 1.8 wt.-%, most preferably at least 1.9 wt.-%, and in particular at least 2.0 wt.-%, relative to the total weight of the dosage form.
Clause 32: The dosage form according to clause 30 or 31, wherein the weight content of the at least one intragranular lubricant is at most 30 wt.-%, preferably at most 20 wt.-%, more preferably at most 15 wt.-%, still more preferably at most 10 wt.-%, yet more preferably at most 5.0 wt.-%, even more preferably at most 3.0 wt.-%, most preferably at most 2.5 wt.-%, and in particular at most 2.0 wt.-%, relative to the total weight of the dosage form.
Clause 33: The dosage form according to any of clauses 30 to 32, wherein the weight content of the at least one intragranular lubricant is within the range of 30±28 wt.-%, preferably 25±23 wt.-%, more preferably 20±18 wt.-%, still more preferably 15±13 wt.-%, yet more preferably 10±8.0 wt.-%, even more preferably 7.0±5.0 wt.-%, most preferably 5.0±3.0 wt.-%, and in particular 2.0±1.5 wt.-%, relative to the total weight of the dosage form.
Clause 34: The dosage form according to any of the preceding clauses, wherein the intragranular phase comprises at least one intragranular diluent; preferably selected from the group consisting of
   - monosaccharides, disaccharides, and oligosaccharides or their derivatives; preferably selected from lactose, e.g. lactose monohydrate, anhydrous lactose, spray dried and/or granulated lactose, lactose hydrate, partially amorphous lactose, completely amorphous lactose; glucose, fructose, sucrose, dextrates, saccharose, raffinose, trehalose, isomaltose, lactitol, and dextrin;
   - sugar alcohols; preferably selected from xylitol, mannitol, maltitol, isomalt, erythritol, and sorbitol;
   - starch or starch derivatives; preferably corn starch, wheat starch, rice starch, tapioca starch, potato starch, pregelatinized starch, or low moisture pregelatinized starch;
   - silicates; preferably magnesium aluminometasilicate;
   - salts of phosphoric acid; preferably calcium salts of phosphoric acid; preferably selected from calcium hydrogen phosphate anhydrous and calcium hydrogen phosphate hydrate;
   - salts of carbonic acid; preferably calcium carbonate, sodium carbonate, potassium carbonate, calcium hydrogencarbonate, sodium hydrogencarbonate, or potassium hydrogencarbonate;
   - salts of lactic acid; preferably calcium lactate; and
   - mixtures thereof; preferably lactose, more preferably lactose monohydrate.
Clause 35: The dosage form according to clause 34, wherein the weight content of the at least one intragranular diluent is at least 10 wt.-%, preferably at least 20 wt.-%, more preferably at least 25 wt.-%, still more preferably at least 30 wt.-%, yet more preferably at least 32 wt.-%, even more preferably at least 34 wt.-%, most preferably at least 36 wt.-%, and in particular at least 38 wt.-%, relative to the total weight of the dosage form.
Clause 36: The dosage form according to clause 34 or 35, wherein the weight content of the at least one intragranular diluent is at most 70 wt.-%, preferably at most 65 wt.-%, more preferably at most 60 wt.-%, still more preferably at most 55 wt.-%, yet more preferably at most 50 wt.-%, even more preferably at most 45 wt.-%, most preferably at most 40 wt.-%, and in particular at most 35 wt.-%, relative to the total weight of the dosage form.
Clause 37: The dosage form according to any of clauses 34 to 36, wherein the weight content of the at least one intragranular diluent is within the range of 70±30 wt.-%, preferably 65±35 wt.-%, more preferably 60±40 wt.-%, still more preferably 55±30 wt.-%, yet more preferably 50±25 wt.-%, even more preferably 45±20 wt.-%, most preferably 40±15 wt.-%, and in particular 35±5.0 wt.-%, relative to the total weight of the dosage form.
Clause 38: The dosage form according to any of the preceding clauses, wherein the intragranular phase comprises at least one intragranular disintegrant; preferably selected from the group consisting of
   - crospovidone;
   - starch and starch derivatives; preferably corn starch, native starch, pregelatinized starch, sodium starch glycollate, hydroxypropyl starch, or carboxymethyl starch;
   - heteroglycanes; preferably alginic acid, sodium alginate, calcium alginate, agar, or guar gum;
   - glucosamines; preferably chitosan;
   - polacrilin potassium or docusate sodium; and
   - mixtures thereof; preferably starch, more preferably corn starch.
Clause 39: The dosage form according to clause 38, wherein the weight content of the at least one intragranular disintegrant is at least 1.0 wt.-%, preferably at least 2.0 wt.-%, more preferably at least 3.0 wt.-%, still more preferably at least 4.0 wt.-%, yet more preferably at least 5.0 wt.-%, even more preferably at least 6.0 wt.-%, most preferably at least 7.0 wt.-%, and in particular at least 8.0 wt.-%, relative to the total weight of the dosage form.
Clause 40: The dosage form according to clause 38 or 39, wherein the weight content of the at least one intragranular disintegrant is at most 40 wt.-%, preferably at most 35 wt.-%, more preferably at most 30 wt.-%, still more preferably at most 25 wt.-%, yet more preferably at most 20 wt.-%, even more preferably at most 15 wt.-%, most preferably at most 12 wt.-%, and in particular at most 10 wt.-%, relative to the total weight of the dosage form.
Clause 41: The dosage form according to any of clauses 38 to 40, wherein the weight content of the at least one intragranular disintegrant is within the range of 30±28 wt.-%, preferably 25±23 wt.-%, more preferably 20±18 wt.-%, still more preferably 17±13 wt.-%, yet more preferably 15±8.0 wt.-%, even more preferably 13±6.0 wt.-%, most preferably 11±4.0 wt.-%, and in particular 9.0±2.0 wt.-%, relative to the total weight of the dosage form.
Clause 42: The dosage form according to any of the preceding clauses, wherein the extragranular phase comprises at least one extragranular diluent; preferably selected from the group consisting of
   - sugar alcohols; preferably selected from xylitol, mannitol, maltitol, isomalt, erythritol, and sorbitol;
   - starch or starch derivatives; preferably corn starch, wheat starch, rice starch, tapioca starch, potato starch, pregelatinized starch, or low moisture pregelatinized starch;
   - cellulose or cellulose derivatives; preferably powdered cellulose, microcrystalline cellulose, or silicified microcrystalline cellulose;
   - silicates; preferably magnesium aluminometasilicate;
   - salts of phosphoric acid; preferably calcium salts of phosphoric acid; preferably selected from calcium hydrogen phosphate anhydrous and calcium hydrogen phosphate hydrate;
   - salts of carbonic acid; preferably calcium carbonate, sodium carbonate, potassium carbonate, calcium hydrogencarbonate, sodium hydrogencarbonate, or potassium hydrogen-carbonate;
   - salts of lactic acid; preferably calcium lactate; and
   - mixtures thereof; preferably cellulose or cellulose derivatives, more preferably microcrystalline cellulose.
Clause 43: The dosage form according to clause 42, wherein the weight content of the at least one extragranular diluent is at least 10 wt.-%, preferably at least 20 wt.-%, more preferably at least 25 wt.-%, still more preferably at least 30 wt.-%, yet more preferably at least 32 wt.-%, even more preferably at least 36 wt.-%, most preferably at least 38 wt.-%, and in particular at least 40 wt.-%, relative to the total weight of the dosage form.
Clause 44: The dosage form according to clause 42 or 43, wherein the weight content of the at least one extragranular diluent is at most 75 wt.-%, preferably at most 70 wt.-%, more preferably at most 65 wt.-%, still more preferably at most 60 wt.-%, yet more preferably at most 55 wt.-%, even more preferably at most 50 wt.-%, most preferably at most 45 wt.-%, and in particular at most 40 wt.-%, relative to the total weight of the dosage form.
Clause 45: The dosage form according to any of clauses 42 to 44, wherein the weight content of the at least one extragranular diluent is within the range of 70±30 wt.-%, preferably 65±35 wt.-%, more preferably 60±40 wt.-%, still more preferably 55±30 wt.-%, yet more preferably 50±25 wt.-%, even more preferably 45±20 wt.-%, most preferably 40±10 wt.-%, and in particular 35±5.0 wt.-%, relative to the total weight of the dosage form.
Clause 46: The dosage form according to any of clauses 42 to 45, wherein the weight content of the at least one extragranular diluent is at least 60 wt.-%, preferably at least 65 wt.-%, more preferably at least 70 wt.-%, still more preferably at least 75 wt.-%, yet more preferably at least 80 wt.-%, even more preferably at least 84 wt.-%, most preferably at least 86 wt.-%, and in particular at least 88 wt.-%, relative to the total weight of the extragranular phase.
Clause 47: The dosage form according to any of clauses 42 to 46, wherein the weight content of the at least one extragranular diluent is at most 99 wt.-%, preferably at most 98 wt.-%, more preferably at most 96 wt.-%, still more preferably at most 94 wt.-%, yet more preferably at most 92 wt.-%, even more preferably at most 90 wt.-%, most preferably at most 88 wt.-%, and in particular at most 86 wt.-%, relative to the total weight of the extragranular phase.
Clause 48: The dosage form according to any of clauses 42 to 47, wherein the weight content of the at least one extragranular diluent is within the range of 60±35 wt.-%, preferably 65±30 wt.-%, more preferably 70±20 wt.-%, still more preferably 75±15 wt.-%, yet more preferably 80±10 wt.-%, even more preferably 82±7.0 wt.-%, most preferably 84±5.0 wt.-%, and in particular 86±3.0 wt.-%, relative to the total weight of the extragranular phase.
Clause 49: The dosage form according to any of clauses 42 to 45, wherein the at least one extragranular diluent has an average particle size of at least 10 µm, preferably at least 20 µm, more preferably at least 30 µm, still more preferably at least 40 µm, and yet more preferably at least 50 µm; preferably determined in accordance with Ph. Eur., 2.9.31: Particle Size Analysis by Laser Light Diffraction.
Clause 50: The dosage form according to any of clauses 42 to 46, wherein the at least one extragranular diluent has an average particle size of at most 200 µm, preferably at most 180 µm, more preferably at most 160 µm, still more preferably at most 140 µm, yet more preferably at most 120 µm, and even more preferably at most 100 µm; preferably determined in accordance with Ph. Eur., 2.9.31: Particle Size Analysis by Laser Light Diffraction.
Clause 51: The dosage form according to any of clauses 42 to 47, wherein the at least one extragranular diluent has an average particle size within the range of 100±90 µm, preferably 100±80 µm, more preferably 100±70 µm, still more preferably 100±60 µm, yet more preferably 100±50 µm, even more preferably 100±40 µm, or 50±40 µm, most preferably 100±30 µm, or 50±30 µm; preferably determined in accordance with Ph. Eur., 2.9.31: Particle Size Analysis by Laser Light Diffraction.
Clause 52: The dosage form according to any of the preceding clauses, wherein the extragranular phase comprises at least one extragranular disintegrant; preferably selected from the group consisting of
   - crospovidone;
   - cellulose or cellulose derivatives; methylcellulose, sodium salts of carboxymethyl cellulose, calcium salts of carboxymethyl cellulose, or cross-linked carboxymethylcellulose, e.g. croscarmellose sodium and/or croscarmellose calcium;
   - heteroglycanes; preferably alginic acid, sodium alginate, calcium alginate, agar, or guar gum;
   - glucosamines; preferably chitosan;
   - polacrilin potassium or docusate sodium; and
   - mixtures thereof; preferably cellulose or cellulose derivatives, more preferably croscarmellose calcium.
Clause 53: The dosage form according to clause 52, wherein the weight content of the at least one extragranular disintegrant is at least 0.2 wt.-%, preferably at least 0.6 wt.-%, more preferably at least 1.0 wt.-%, still more preferably at least 1.4 wt.-%, yet more preferably at least 1.8 wt.-%, even more preferably at least 2.2 wt.-%, most preferably at least 2.6 wt.-%, and in particular at least 3.0 wt.-%, relative to the total weight of the dosage form.
Clause 54: The dosage form according to any clause 52 or 53, wherein the weight content of the at least one extragranular disintegrant is at most 8.0 wt.-%, preferably at most 7.0 wt.-%, more preferably at most 6.0 wt.-%, still more preferably at most 5.0 wt.-%, yet more preferably at most 4.0 wt.-%, even more preferably at most 3.0 wt.-%, most preferably at most 2.5 wt.-%, and in particular at most 2.0 wt.-%, relative to the total weight of the dosage form.
Clause 55: The dosage form according to any of clauses 52 to 54, wherein the weight content of the at least one extragranular disintegrant is within the range of 10±9.0 wt.-%, preferably 8.0±7.0 wt.-%, more preferably 7.0±6.0 wt.-%, still more preferably 6.0±5.0 wt.-%, yet more preferably 5.0±4.0 wt.-%, even more preferably 4.0±3.0 wt.-%, most preferably 3.0±2.0 wt.-%, and in particular 2.5±1.0 wt.-%, relative to the total weight of the dosage form.
Clause 56: The dosage form according to any of the preceding clauses, wherein the extragranular phase comprises at least one extragranular glidant; preferably selected from the group consisting of colloidal silica, talc, magnesium trisilicate, and mixtures thereof; preferably colloidal silica, more preferably colloidal anhydrous silica.
Clause 57: The dosage form according to clause 56, wherein the weight content of the at least one extragranular glidant is within the range of 5.0±4.5 wt.-%, preferably 2.0±1.5 wt.-%, more preferably 1.5±1.0 wt.-%, still more preferably 1.0±0.8 wt.-%, yet more preferably 0.8±0.6 wt.-%, even more preferably 0.7±0.5 wt.-%, most preferably 0.6±0.4 wt.-%, and in particular 0.5±0.4 wt.-%, relative to the total weight of the dosage form.
Clause 58: The dosage form according to any of the preceding clauses, wherein the extragranular phase comprises at least one extragranular lubricant; preferably selected from the group consisting of
   - C₁₂₋₂₀-fatty acids and C₁₂₋₂₀-fatty acid esters, preferably selected from glyceryl monostearate, glyceryl tribehenate, and glyceryl dibehenate;
   - metal salts of C₁₂₋₂₀-fatty acids and derivatives thereof; preferably selected from magnesium stearate, calcium stearate, aluminum stearate, zinc stearate, magnesium palmitate, magnesium oleate, and sodium stearyl fumarate;
   - hydrogenated oils, preferably hydrogenated vegetable oil or hydrogenated castor oil;
   - waxes, preferably meads wax or spermaceti;
   - boric acid; and
   - mixtures thereof; preferably magnesium stearate.
Clause 59: The dosage form according to clause 58, wherein the weight content of the at least one extragranular lubricant is within the range 5.0±4.5 wt.-%, preferably 2.0±1.5 wt.-%, more preferably 1.5±1.0 wt.-%, still more preferably 1.0±0.8 wt.-%, yet more preferably 0.8±0.6 wt.-%, even more preferably 0.7±0.5 wt.-%, most preferably 0.6±0.5 wt.-%, and in particular 0.6±0.4 wt.-%, relative to the total weight of the dosage form.
Clause 60: The dosage form according to any of the preceding clauses, which comprises or essentially consists of
   (i) the intragranular phase comprising
      - candesartan cilexetil;
      - optionally, at least one intragranular binder, preferably hydroxypropyl cellulose, more preferably at a weight content within the range of 3.0±2.0 wt.-%;
      - optionally, at least one intragranular lubricant, preferably polyethylene glycol, more preferably at a weight content within the range of 2.0±1.5 wt.-%;
      - at least one intragranular diluent, preferably lactose monohydrate, more preferably at a weight content within the range of 35±10 wt.-%; and
      - at least one intragranular disintegrant, preferably corn starch, more preferably at a weight content within the range of 9.0±4.0 wt.-%; and
   (ii) the extragranular phase comprising
      - indapamide;
      - at least one extragranular diluent, preferably microcrystalline cellulose, more preferably at a weight content within the range of 35±10 wt.-%;
      - optionally, at least one extragranular disintegrant, preferably carmellose calcium, more preferably at a weight content within the range of 2.5±2.0 wt.-%;
      - optionally, at least one extragranular glidant, preferably colloidal anhydrous silica, more preferably at a weight content within the range of 0.5±0.2 wt.-%; and
      - optionally, at least one extragranular lubricant, preferably magnesium stearate, more preferably at a weight content within the range of 0.6±0.2 wt.-%; in each case relative to the total weight of the dosage form.
Clause 61: The dosage form according to any of the preceding clauses, which is compacted or compressed.
Clause 62: The dosage form according to any of the preceding clauses, which is selected from the group consisting of tablets, pills, powders, lozenges, sachets, soft gelatin capsules, hard gelatin capsules, and suppositories; preferably tablets.
Clause 63: The dosage form according to any of the preceding clauses, which is coated; preferably with a water-soluble coating material.
Clause 64: The dosage form according to any of the preceding clauses, wherein the overall weight of the dosage form is within the range of 130±50 mg, preferably 130±40 mg, more preferably 130±30 mg, still more preferably 130±25 mg, yet more preferably 130±20 mg, even more preferably 130±15 mg, most prefer-ably 130±10 mg, and in particular 130±5.0 mg.
Clause 65: The dosage form according to any of the preceding clauses, wherein the acceptance value of candesartan cilexetil is at most 8.0, preferably at most 6.0, more preferably at most 5.0, still more preferably at most 4.5, yet more preferably at most 4.0, even more preferably at most 3.5, most preferably at most 3.0, and in particular at most 2.5; preferably determined according to the content uniformity test in accordance with Ph. Eur.
Clause 66: The dosage form according to any of the preceding clauses, wherein the acceptance value of candesartan cilexetil is within the range of 2.0±0.5, or 2.5±1.0, or 2.5±0.5, or 3.0±1.5, or 3.0±1.0, or 3.0±0.5, or 3.5±2.0, or 3.5±1.5, or 3.5±1.0, or 3.5±0.5, or 4.0±2.5, or 4.0±2.0, or 4.0±1.5, or 4.0±1.0, or 4.0±0.5, or 4.5±3.0, or 4.5±2.5, or 4.5±2.0, or 4.5±1.5, or 4.5±1.0, or 4.5±0.5, or 5.0±3.5, or 5.0±3.0, or 5.0±2.5, or 5.0±2.0, or 5.0±1.5, or 5.0±1.0, or 5.0±0.5; preferably determined according to the content uniformity test in accordance with Ph. Eur.
Clause 67: The dosage form according to any of the preceding clauses, wherein the acceptance value of indapamide is at most 8.0, preferably at most 6.0, more preferably at most 5.0, still more preferably at most 4.5, yet more preferably at most 4.0, even more preferably at most 3.5, most preferably at most 3.0, and in particular at most 2.5; preferably determined according to the content uniformity test in accordance with Ph. Eur.
Clause 68: The dosage form according to any of the preceding clauses, wherein the acceptance value of indapamide is within the range of 2.0±0.5, or 2.5±1.0, or 2.5±0.5, or 3.0±1.5, or 3.0±1.0, or 3.0±0.5, or 3.5±2.0, or 3.5±1.5, or 3.5±1.0, or 3.5±0.5, or 4.0±2.5, or 4.0±2.0, or 4.0±1.5, or 4.0±1.0, or 4.0±0.5, or 4.5±3.0, or 4.5±2.5, or 4.5±2.0, or 4.5±1.5, or 4.5±1.0, or 4.5±0.5, or 5.0±3.5, or 5.0±3.0, or 5.0±2.5, or 5.0±2.0, or 5.0±1.5, or 5.0±1.0, or 5.0±0.5; preferably determined according to the content uniformity test in accordance with Ph. Eur.
Clause 69: The dosage form according to any of the preceding clauses, wherein the content of indapamide impurity A is at most 10 ppm, preferably at most 6.0 ppm, more preferably at most 4.0 ppm, still more preferably at most 3.5 ppm, yet more preferably at most 3.0 ppm, even more preferably at most 2.5 ppm, most preferably at most 2.0 ppm, and in particular at most 1.5 ppm; preferably after storage in aluminum - PVDC/PVC 250/90 blisters for 3 months under conditions of 40°C/75%.
Clause 70: The dosage form according to any of the preceding clauses for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supra-ventricular and ventricular arrhythmias, atrial fibrillation, atrial flutter, detrimental vascular re-modeling, myocardial infarction and its sequelae, atherosclerosis, angina (whether unstable or stable), renal insufficiency (diabetic and non-diabetic), angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.
Clause 71: A process for the preparation of a dosage form according to any of the preceding clauses.
Clause 72: The process according to clause 71, which comprises the steps of:
   (a) providing a granulation liquid comprising
      - candesartan cilexetil;
      - one or more solvents, preferably water; and
      - optionally, one or more excipients, preferably at least one intragranular binder as defined in any of clauses 26 to 29 and/or at least one intragranular lubricant as defined in any of clauses 30 to 33;
   (b) providing a granulation mixture comprising one or more excipients, preferably at least one intragranular diluent as defined in any of clauses 34 to 37 and/or at least one intragranular disintegrant as defined in any of clauses 38 to 41;
   (c) wet granulating the granulation mixture provided in step (b) with the granulation liquid provided in step (a) thereby obtaining an intragranular phase;
   (d) mixing the intragranular phase obtained in step (c) with indapamide and optionally one or more excipients, preferably at least one extragranular diluent as defined in any of clauses 42 to 51, at least one extragranular disintegrant as defined in any of clauses 52 to 55, at least one extragranular glidant as defined in clause 56 or 57, and/or at least one extragranular lubricant as defined in clause 58 to 59, thereby obtaining a compression mixture which comprises the intragranular phase obtained in step (c) and an extragranular phase comprising the indapamide and the optionally present one or more excipients, preferably at least one extragranular diluent, at least one extragranular disintegrant, at least one extragranular glidant, and/or at least one extragranular lubricant;
   (e) optionally, compressing the compression mixture provided in step (d) by means of a tablet press thereby obtaining a tablet.
Clause 73: The process according to clause 71 or 72, wherein in step (c) the wet granulation is performed by means of a granulating device; preferably a high shear mixer or a fluid bed dryer.
Clause 74: The process according to any of clauses 71 to 73, wherein step (c) involves drying and/or sieving of the intragranular phase thereby obtaining a dried and/or sieved intragranular phase.
Clause 75: The process according to any of clauses 71 to 74, wherein in step (d) the mixing is performed by means of a mixing device; preferably a container mixer.
Clause 76: A pharmaceutical dosage form obtainable by the process according to any of clauses 71 to 75.

The following examples further illustrate the invention but are not to be construed as limiting its scope.

### Preparation of pharmaceutical dosage forms:

The following formulations were prepared:

| [wt.-%] | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 |
|---|---|---|---|---|---|---|---|---|---|---|---|---|---|---|---|---|
| Batch size | | 5,000 | 22,000 | 22,000 | 22,000 | 15,000 | 15,000 | 30,000 | 200,000 | 200,000 | 36,500 | 37,000 | 200,000 | 200,000 | 200,000 | 200,000 |

| *Intragranular* | | | | | | | | | | | | | | | | |
|---|---|---|---|---|---|---|---|---|---|---|---|---|---|---|---|---|
| Candesartan cilexetil | active ingredient | 2.42 | 9.39 | 12.27 | 6.15 | 9.70 | 2.42 | 12.31 | 12.31 | 12.31 | 6.15 | 12.12 | 6.35 | 6.15 | 12.31 | 6.15 |
| Hydroxypropyl cellulose | binder | 1.21 | 2.35 | 3.07 | 3.08 | 4.85 | 4.85 | 3.08 | 3.08 | 3.08 | 3.08 | 3.03 | 3.17 | 3.08 | 3.08 | 3.08 |
| Polyethylene glycol 8000 | lubricant | 0.39 | 1.53 | 1.99 | 2.00 | 1.58 | 1.58 | 2.00 | 2.00 | 2.00 | 2.00 | 1.97 | 2.06 | 2.00 | 2.00 | 2.00 |
| Water | granulation solvent | q.s. | q.s. | q.s. | q.s. | q.s. | q.s. | q.s. | q.s. | q.s. | q.s. | q.s. | q.s. | q.s. | q.s. | q.s. |
| Lactose monohydrate | diluent | 12.26 | 46.58 | 52.81 | 67.23 | 49.05 | 56.33 | 42.29 | 31.75 | 33.29 | 31.77 | 31.27 | 37.54 | 39.46 | 33.29 | 39.46 |
| Corn starch | disintegrant | 3.03 | 11.74 | 15.34 | 15.38 | 12.12 | 12.12 | 12.31 | 9.23 | 8.46 | 9.23 | 9.09 | 8.73 | 8.46 | 8.46 | 8.46 |
| Iron oxide red | coloring agent | 0.01 | 0.02 | 0.02 | | 0.04 | 0.04 | 0.02 | 0.02 | 0.02 | | 0.02 | | | 0.02 | |

| *Extragranular* | | | | | | | | | | | | | | | | |
|---|---|---|---|---|---|---|---|---|---|---|---|---|---|---|---|---|
| Indapamide | active ingredient | 0.76 | 1.47 | 1.92 | 1.92 | 0.76 | 0.76 | 1.95 | 1.96 | 1.96 | 1.96 | 1.93 | 2.02 | 1.96 | 1.96 | 1.96 |
| Microcrystalline cellulose | diluent | 77.36 | 23.47 | 6.04 | | 19.64 | 19.64 | 22.97 | 36.58 | 36.23 | 41.19 | 36.02 | 37.38 | 36.23 | 35.85 | 35.85 |
| Carmellose calcium | disintegrant | 1.52 | 2.99 | 3.91 | 3.92 | 1.52 | 1.52 | 1.96 | 1.96 | 1.54 | 3.50 | 3.45 | 1.59 | 1.54 | 1.92 | 1.92 |
| Silica, colloidal anhydrous | glidant | 0.48 | | | | 0.45 | 0.45 | 0.50 | 0.50 | 0.50 | 0.50 | 0.49 | 0.52 | 0.50 | 0.50 | 0.50 |
| Magnesium stearate | lubricant | 0.55 | 0.47 | 0.61 | 0.31 | 0.30 | 0.30 | 0.62 | 0.62 | 0.62 | 0.62 | 0.61 | 0.63 | 0.62 | 0.62 | 0.62 |
| Tablet | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |

The grade of microcrystalline cellulose that was used in the examples had a nominal mean particle size of 50 µm.

A dispersion of candesartan cilexetil, hydroxypropyl cellulose and polyethylene glycol 8000 in water was prepared.

Lactose monohydrate, corn starch and iron oxide red were sieved and mixed in a fluid bed granulator. The mixture was granulated with the dispersion of candesartan cilexetil, hydroxypropyl cellulose and polyethylene glycol 8000 in water by use of a fluid bed granulator.

After granulation, the granulate was dried and sieved. The dried granulate was mixed with indapamide, microcrystalline cellulose, carmellose calcium and colloidal anhydrous silica in a mixing container. The mixture was lubricated with magnesium stearate in a mixing container to obtain a compression mixture. The compression mixture was compressed into tablets.

### Uniformity of pharmaceutical dosage forms:

To test the consistency of content of both active ingredients in the final product, uniformity of the dosage units was demonstrated by the content uniformity test. Samples from Examples 1-13 were tested and acceptance value (AV) for candesartan cilexetil and indapamide calculated. The number of samples that were analyzed depend on the size of the batch. For smaller batches (Examples 3 and 4) a single sample representing the whole batch was analyzed. For larger batches (Examples 1, 2, 5, and 6) additional samples representing different time points in the tableting process were also analyzed. The results are shown in the table here below:

| | | AV candesartan | AV indapamide |
|---|---|---|---|
| Ex. 1 | Representative | Not tested | |
| Ex. 2 | Representative | 2.6 | 4.7 |
| Ex. 3 | Representative | 1.6 | 5.7 |
| Ex. 4 | Representative | 2.3 | 6.7 |
| Ex. 5 | Representative | Not tested | |
| Ex. 6 | Representative | Not tested | |
| Ex. 7 | Representative | 1.1 | 2.5 |
| Ex. 8 | Representative | 3.1 | 4.2 |
| | Beginning | 6 | 8.8 |
| | Middle | 1.5 | 4.6 |
| | End | 4 | 6.9 |
| Ex. 9 | Beginning | 6.3 | 4.8 |
| | Middle | 3.6 | 3.6 |
| | End | 3.9 | 3.1 |
| Ex. 10 | Representative | 2 | 2 |
| Ex. 11 | Representative | 2.2 | 2.4 |
| Ex. 12 | Beginning | 4 | 4.4 |
| | Middle | 3.5 | 4.1 |
| Ex. 13 | Beginning | 3.9 | 2.2 |
| | Middle | 3.7 | 2.4 |

In the above table, "representative" refers a sample composed of tablets that are taken at different time points including but not limited to tablets taken at the beginning, in the middle or at the end of the process. Tablets were mixed so as to be as close to an average tablet as possible. Representative samples were analyzed separately.

By adjusting the composition of the blend and incorporating a large portion of candesartan granulate in it, settling down of indapamide particles can be prevented and a good processability of the blend ensured (good flowability, undemanding parameters for the process, low acceptance values (AV) of tablets).

Proper uniformity of indapamide was surprisingly achieved even without the use of wet or dry granulation of indapamide. It was found that the use of a high amount of an extragranular diluent such as microcrystalline cellulose, preferably with a small particle size, is suitable for achieving this feature.

### Impurities of indapamide:

Indapamide impurities were also tested. The tablets of Example 3 were packed into aluminum - PVDC/PVC 250/90 blisters and exposed to 40°C/75% conditions. After 3 months, the results showed no significant increase in indapamide A impurity. The results are shown in the table here below:

| | Time (months) | Impurity A (ppm) |
|---|---|---|
| Ex. 3 | 0 | 0.96 |
| | 3 | 1.2 |

Indapamide was admixed into the dry phase of candesartan cilexetil granulate. To ensure uniformity of indapamide in the blend, an extragranular diluent such as microcrystalline cellulose, preferably with a small particle size, can be added. This way of adding indapamide enables its stability even in relatively permeable packaging, such as blister from PVC/PVDC 250/90 foil.

## Claims

1. A solid pharmaceutical dosage form for oral administration comprising
(i) an intragranular phase comprising candesartan cilexetil; and
(ii) an extragranular phase comprising indapamide.

2. The dosage form according to claim 1, wherein essentially the total amount of the indapamide that is contained in the dosage form is present in the extragranular phase.

3. The dosage form according to claim 1 or 2, wherein the extragranular phase comprises at least one extragranular diluent; preferably selected from the group consisting of
- sugar alcohols; preferably selected from xylitol, mannitol, maltitol, isomalt, erythritol, and sorbitol;
- starch or starch derivatives; preferably corn starch, wheat starch, rice starch, tapioca starch, potato starch, pregelatinized starch, or low moisture pregelatinized starch;
- cellulose or cellulose derivatives; preferably powdered cellulose, microcrystalline cellulose, or silicified microcrystalline cellulose;
- silicates; preferably magnesium aluminometasilicate;
- salts of phosphoric acid; preferably calcium salts of phosphoric acid; preferably selected from calcium hydrogen phosphate anhydrous and calcium hydrogen phosphate hydrate;
- salts of carbonic acid; preferably calcium carbonate, sodium carbonate, potassium car-bonate, calcium hydrogencarbonate, sodium hydrogencarbonate, or potassium hydrogen-carbonate;
- salts of lactic acid; preferably calcium lactate; and
- mixtures thereof;
preferably cellulose or cellulose derivatives, more preferably microcrystalline cellulose.

4. The dosage form according to claim 3, wherein the weight content of the at least one extragranular diluent is at least 10 wt.-%, preferably at least 20 wt.-%, more preferably at least 25 wt.-%, still more preferably at least 30 wt.-%, yet more preferably at least 32 wt.-%, even more preferably at least 36 wt.-%, most preferably at least 38 wt.-%, and in particular at least 40 wt.-%, relative to the total weight of the dosage form.

5. The dosage form according to any of the preceding claims, which provides conventional release of candesartan.

6. The dosage form according to any of the preceding claims, wherein the weight content of candesartan cilexetil is within the range of 21±16 wt.-%, preferably 19±14 wt.-%, more preferably 17±12 wt.-%, still more preferably 15±10 wt.-%, yet more preferably 13±8.0 wt.-%, even more preferably 11±6.0 wt.-%, most preferably 9.0±4.0 wt.-%, and in particular 6.0±2.0 wt.-%, or 12±2.0 wt.-%, relative to the total weight of the dosage form.

7. The dosage form according to any of the preceding claims, wherein the intragranular phase is prepared by wet granulation; preferably aqueous wet granulation; more preferably wherein the candesartan cilexetil is dissolved in the granulation liquid.

8. The dosage form according to any of the preceding claims, which provides conventional release of indapamide.

9. The dosage form according to any of the preceding claims, wherein the weight content of indapamide is within the range of 4.0±3.5 wt.-%, preferably 3.5±3.0 wt.-%, more preferably 3.0±2.5 wt.-%, still more preferably 2.8±2.3 wt.-%, yet more preferably 2.6±2.0 wt.-%, even more preferably 2.4±1.5 wt.-%, most preferably 2.2±1.0 wt.-%, and in particular 2.0±0.5 wt.-%, relative to the total weight of the dosage form.

10. The dosage form according to any of the preceding claims, wherein the relative weight ratio of the extragranular phase to the intragranular phase is within the range of from 1.0:1.0 to 1.0:2.1, preferably from 1.0:1.0 to 1.0:1.9, more preferably from 1.0:1.0 to 1.0:1.7, still more preferably from 1.0:1.0 to 1.0:1.5, and yet more preferably from 1.0:1.0 to 1.0:1.3.

11. The dosage form according to any of the preceding claims, wherein the acceptance value of candesartan cilexetil is at most 8.0, preferably at most 6.0, more preferably at most 5.0, still more preferably at most 4.5, yet more preferably at most 4.0, even more preferably at most 3.5, most preferably at most 3.0, and in particular at most 2.5; preferably determined according to the content uniformity test in accordance with Ph. Eur.

12. The dosage form according to any of the preceding claims, wherein the acceptance value of indapamide is at most 8.0, preferably at most 6.0, more preferably at most 5.0, still more preferably at most 4.5, yet more preferably at most 4.0, even more preferably at most 3.5, most preferably at most 3.0, and in particular at most 2.5; preferably determined according to the content uniformity test in accordance with Ph. Eur.

13. The dosage form according to any of the preceding claims, wherein the content of indapamide impurity A is at most 10 ppm, preferably at most 6.0 ppm, more preferably at most 4.0 ppm, still more preferably at most 3.5 ppm, yet more preferably at most 3.0 ppm, even more preferably at most 2.5 ppm, most preferably at most 2.0 ppm, and in particular at most 1.5 ppm; preferably after storage in aluminum - PVDC/PVC 250/90 blisters for 3 months under conditions of 40°C/75%.

14. The dosage form according to any of the preceding claims for use in the treatment or prevention of a condition or disease selected from the group consisting of hypertension, heart failure, such as (acute and chronic) congestive heart failure, left ventricular dysfunction and hypertrophic cardiomyopathy, diabetic cardiac myopathy, supra-ventricular and ventricular arrhythmias, atrial fibrillation, atrial flutter, detrimental vascular re-modeling, myocardial infarction and its sequelae, atherosclerosis, angina (whether unstable or stable), renal insufficiency (diabetic and non-diabetic), angina pectoris, diabetes, secondary aldosteronism, primary and secondary pulmonary hypertension, renal failure conditions, such as diabetic nephropathy, glomerulonephritis, scleroderma, glomerular sclerosis, proteinuria of primary renal disease, and also renal vascular hypertension, diabetic retinopathy, the management of other vascular disorders, such as migraine, peripheral vascular disease, Raynaud's disease, luminal hyperplasia, cognitive dysfunction, such as Alzheimer's, glaucoma and stroke; preferably hypertension and heart failure.

15. A process for the preparation of a dosage form according to any of the preceding claims, the process comprising the steps of:
(a) providing a granulation liquid comprising
- candesartan cilexetil;
- one or more solvents, preferably water; and
- optionally, one or more excipients, preferably at least one intragranular binder and/or at least one intragranular lubricant;
(b) providing a granulation mixture comprising one or more excipients, preferably at least one intragranular diluent and/or at least one intragranular disintegrant;
(c) wet granulating the granulation mixture provided in step (b) with the granulation liquid provided in step (a) thereby obtaining an intragranular phase;
(d) mixing the intragranular phase obtained in step (c) with indapamide and optionally one or more excipients, preferably at least one extragranular diluent, at least one extragranular disintegrant, at least one extragranular glidant, and/or at least one extragranular lubricant, thereby obtaining a compression mixture which comprises the intragranular phase obtained in step (c) and an extragranular phase comprising the indapamide and the optionally present one or more excipients, preferably at least one extragranular diluent, at least one extragranular disintegrant, at least one extragranular glidant, and/or at least one extragranular lubricant;
(e) optionally, compressing the compression mixture provided in step (d) by means of a tablet press thereby obtaining a tablet.
